(19) **Europäisches Patentamt / European Patent Office / Office européen des brevets**

(11) **EP 4 632 373 A1**

(12) **EUROPEAN PATENT APPLICATION**
published in accordance with Art. 153(4) EPC

(43) Date of publication:
**15.10.2025  Bulletin 2025/42**

(21) Application number: **22936067.2**

(22) Date of filing: **05.12.2022**

(51) International Patent Classification (IPC):
**G01N 33/18** (2006.01)  **C02F 9/00** (2023.01)

(52) Cooperative Patent Classification (CPC):
**G01N 33/18; C02F 1/008; G01N 33/1826;**
**C02F 2209/006**

(86) International application number:
**PCT/ES2022/070777**

(87) International publication number:
**WO 2024/121441 (13.06.2024 Gazette 2024/24)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **UNIVERSIDAD DE NAVARRA ESCUELA DE INGENIERIA (TECNUN)**
**20018 San Sebastian Guipuzcoa (ES)**

(72) Inventors:
• **AYESA ITURRATE, Eduardo José**
  **20018 SAN SEBASTIAN (GIPUZKOA) (ES)**
• **FERNANDEZ AREVALO, Tamara**
  **20018 SAN SEBASTIAN (GIPUZKOA) (ES)**
• **INSAUSTI SARASOLA, Xabier**
  **20018 SAN SEBASTIAN (GIPUZKOA) (ES)**
• **GUTIERREZ GUTIERREZ, Jesús**
  **20018 SAN SEBASTIAN (GIPUZKOA) (ES)**

(74) Representative: **Evens, Paul Jonathan et al**
**Maguire Boss**
**24 East Street**
**St. Ives, Cambridgeshire PE27 5PD (GB)**

(54) **METHOD FOR THE QUALITATIVE DETERMINATION OF WATER QUALITY**

(57)   The invention relates to a method for determining whether water quality is suitable for subsequent use, comprising the steps of:
- measuring a plurality of quality parameters of different water samples at different time points, obtaining a matrix of measured values of parameters for the different time points;
- processing the matrix of measured values to obtain a matrix of processed values such that the difference between said matrices is minimal with the condition that the value of the rank of the matrix of processed values approximates or is equal to a predetermined value less than the rank of the matrix of measured values, the rank of the matrix of processed values preferably being equal to 2 or 3;
- determining water quality by comparing values from the matrix of processed values with predetermined acceptance thresholds for water quality parameters.

Fig. 3

EP 4 632 373 A1

## Description

### Sector of the art

The present invention belongs to the field of water quality, more specifically to the field of the qualitative determination of water quality, and relates to a method for measuring water quality parameters and the subsequent processing of said parameters, may also comprise a screening of the processed quality parameters, all this to obtain more precise water quality parameters. The invention also relates to the use of said method to increase efficiency in the use of water, and for the optimization and calibration of WWTPs (wastewater treatment plants).

### Prior Art

Efficiency in the consumption of a resource as precious as water is very important given that it is an essential and sometimes scarce resource. On the one hand, humans need water to drink and carry out their activities, and on the other hand it is a precious resource in all types of industries, for example in the food industry where the efficient use of water marks its competitiveness. This is why the industry requires efforts to further minimize the use of water used, especially to produce food and beverages, guaranteeing its most efficient use and purification. In fact, 70% of global freshwater consumption occurs within the food industry (Food and Agriculture Organization of the United Nations, FAO, 2019).

One of the main reasons for the high consumption of water in the food industry is the poor monitoring and control of water requirements by the processes. Therefore, it is a necessity to have industrial water flows identified and characterized, in order to know the degree of optimization of the system or the possible points of improvement and action.

A second important reason is the low application of water reuse techniques. The reuse of water within the sector is conditional on the availability of regeneration or conditioning systems adapted to the characteristics of the water to be reused, and for the economic cost of said systems. Due to these limitations, the percentage of water reuse in food industries is only 2.4%, a very low figure even compared to that of the manufacturing industry, which is also low, which stands at 8.9% (data prepared from the Survey on water use in the industrial sector, INE 1999). It is for this reason that there is a need to identify water flows with potential for reuse, which in turn will reduce costs derived from managing the water cycle.

At present in the prior art, in order to determine water quality and thus identify the water flows that can be reused, physical-chemical parameters characteristic of water quality are measured using measurement systems (for example parameters such as chemical or biological oxygen demand, volatile and total suspended solids, ammonium, nitrogen, phosphate, etc.), and by direct comparison of the measured values with known theoretical threshold values of the water that identify it as being of sufficient quality or not, said water flow is reused or rejected. Other known simple and non-rigorous methods of the prior art attempt to predict water quality by simple linear interpolation between existing measured parameters.

Nevertheless, said methods of the prior art based on pure comparison and extrapolation of water quality parameters end up rejecting many water flows that could be reusable with a more exhaustive method of water quality control. Furthermore, in the case of making errors in the measurement of said parameters (parameters missing for the measurement or measured incorrectly), these parameters cannot be used to compare with threshold values or to extrapolate or will lead to considerable errors if used, with which the reliability of said methods of determining water quality is compromised.

For this reason, the field of water quality needs more exhaustive water control methodologies, that allow the reuse of water flows that are unnecessarily wasted with the methodologies known until now, thereby increasing efficiency in water use and reducing water management costs in industries and treatment plants, among others.

The applicant of the present invention has also observed that for water quality parameters (for example measured in WWTPs: wastewater treatment plants), it does not always mean that because a measurement deviates from the mean or exceeds a predetermined threshold, that the water flow with said parameter does not have sufficient quality and must be rejected. This is why simple comparison and extrapolation models end up rejecting water flows of sufficient quality.

To solve the problems previously outlined in the measurement and control of water quality, and to provide a more reliable method to determine water quality allowing for greater reuse, the applicant proposes a procedure for systematic measurement and processing of measured water data detailed below.

### Object of the invention

The object of the present invention is a method for determining water quality. Specifically, after measuring the water quality parameters, said data is agglomerated in a matrix of measured values that is processed to obtain a matrix of processed values, by decreasing the rank of the matrix of processed values with respect to the matrix of measured values

to a value approximating or equal to a predetermined rank, preferably a rank of 2 or 3, while minimizing the resulting difference between the matrix of processed values and the matrix of measured values. This allows both the reconstruction of those values of the missing measured quality parameters, such as processing those measured values to more precise parameter values of the water quality.

Optionally, a step of screening the matrix of processed values can be included, in order to identify and eliminate missing or erroneous values, that is, those values that exceed a predetermined error threshold, and that caused an increase in the rank of the matrix of values processed before screening. Subsequently, the matrix of screened values can be processed to obtain a matrix of reprocessed values, by decreasing the rank of the matrix of screened values with respect to the matrix of screened values, while minimizing the resulting difference between the matrix of reprocessed values and the matrix of screened values. This results in a matrix of more precise reprocessed values, which allows the quality of the water to be determined even more precisely.

Minimizing the rank of the matrix of measured values or the matrix of screened values to a value approximating or equal to a predetermined rank can be carried out by matrix completion algorithms, such as algorithms based on the minimization of the rank or the nuclear norm of the matrix.

In the previous matrices as a non-limiting example, the rows correspond to each measurement made of the quality parameters of a water sample (measurement time point), while the columns refer to the water quality parameters measured in the water sample (chemical oxygen demand, suspended solids, phosphates, ammonium, etc.); or vice versa. Although the matrices could also comprise any other type of parameters or measurements related to water quality samples, organized in rows or columns.

All these previous steps allow errors in the quality parameters of the measured water to be minimized, errors that may have been caused by a multitude of reasons. Examples of these errors can be failures in the measurement sensors, under the conditions of the measured water sample itself, human errors, or errors due to missing measurement values that have not been measured or have been lost, etc.

In summary, the methodology of the present invention combines data acquisition/measurement phases, and processing these mathematically, to optimize precision in determining water quality. All of this allows errors caused by erroneous (spurious) or missing quality parameter values to be minimized, with the consequent improvement in the estimation of water quality and the efficiency of its use.

The method of determining water quality (for example for discharge, purification, industrial use, consumption, etc.) of the present invention, to determine whether it is suitable for subsequent use, comprises the following steps:

- measuring a plurality of quality parameters of different water samples at different time points, obtaining a matrix of measured values of parameters for the different time points;
- processing the matrix of measured values to obtain a matrix of processed values such that the difference between said matrices is minimal with the condition that the value of the rank of the matrix of processed values approximates or is equal to a predetermined value less than the rank of the matrix of measured values, the rank of the matrix of processed values preferably being equal to 2 or 3;
- determining water quality by comparing values from the matrix of processed values with predetermined acceptance thresholds for water quality parameters.

A second optional aspect of the invention relates to additionally carrying out the following steps:

- screening the matrix of processed values, screening the values of the matrix such that the difference between the processed values and the measured values, or said difference with respect to the corresponding measured values, exceeds a predetermined error;
- reprocessing the matrix of screened values to obtain a matrix of reprocessed values such that the difference between said matrices is minimal with the condition that the value of the rank of the matrix of reprocessed values approximates or is equal to a predetermined value less than the rank of the matrix of screened values, the rank of the matrix of processed values preferably being equal to 2 or 3;
and
- determining water quality by comparing values from the matrix of reprocessed values, instead of the matrix of processed values, with predetermined acceptance thresholds for water quality parameters.

A third optional aspect of the invention comprises using a matrix completion algorithm to process the matrix of measured values or the matrix of screened values, which is an algorithm based on the minimization of the rank or nuclear norm of the matrix.

A fourth optional aspect of the invention comprises establishing as the predetermined rank of the matrix of processed or reprocessed values, the rank of a matrix of measured values for a reference water sample measured at different time points. This means that the rank to be predetermined can be calibrated for the resulting matrix of processed or reprocessed values.

A fifth optional aspect of the invention comprises in the matrix of screened values eliminating those measurements of water samples whose measured values contain a number of screened values greater than 50% of the number of total parameters of the measurement of the water sample, preferably greater than 90%, or eliminating those measurements from water samples that do not have at least 2 non-screened values. The screened values are the values that are screened or discarded from the matrix of screened values, that is, the values identified as erroneous or missing that are discarded from the matrix of screened values.

Screened values are understood as values identified as erroneous or missing that are discarded from the matrix of screened values. Acceptable values, on the other hand, are understood as non-screened values.

A sixth optional aspect of the invention comprises, before processing the matrix of measured values or reprocessing the matrix of screened values, normalizing the values of the matrix to values that are between 0 and 1.

A seventh aspect of the invention relates to the use of the method of the previous aspects of the invention for the treatment of wastewater, for example to improve mathematical simulation models for optimization and calibration of WWTPs (wastewater treatment plants).

To process the matrix of measured values or the matrix of screened values, any known matrix completion method can be used, such as using a known matrix completion algorithm (e.g. OptSpace, SVT, FPCA, etc.), for example algorithms based on the minimization of the rank of the matrix or the nuclear norm of the matrix, although the present invention will preferably use the algorithm known as LMaFit both to process the data of the matrix of measured values or the matrix of screened values. This LMaFit algorithm, in the particular case of processing water quality parameters, allows the completion of said missing or erroneous (spurious) parameters of the matrix.

In summary, using this methodology based on the measurement (online or offline) of water quality parameters and their subsequent processing, resolving a data completeness problem, it is possible to determine the water quality more precisely, which in turn allows for better reuse. This contrasts with conventional methods of simple comparison between water quality parameters, that would lead to discarding water that is of sufficient quality and is reusable, thereby increasing efficiency in water use, since water flows are used that would be determined as disposable by conventional water quality control methods.

This solution is particularly useful in this specific scenario of water quality control, since it has been observed that measurements of water quality parameters generate a lot of redundant information that can be compressed or even partially discarded without compromising an efficient determination of water quality.

The applicant of this patent is not aware of prior use of any similar methodology to process and optionally screen series of experimentally measured water quality parameters. Nor is there any evidence that there is another different procedure for the reconstruction of water quality data beyond simple and non-rigorous procedures such as simple linear interpolation between existing data; or the application of empirical ratios between variables.

A particular example of application of the methodology for measuring and processing water quality control parameters according to the invention is provided later in the detailed description of the invention.

Lastly, it is indicated that the commas of the values in this specification (for example those in the cells of Figures 1-5) indicate decimal points.

**Description of the Figures**

Figure 1 visually shows an example of a matrix of measured values, obtained after measuring water quality parameters in a WWTP (wastewater treatment plant). Data missing in the cells of the matrix of measured values are shown with a dash (-) in light gray cells.

Figure 2 shows the normalization (optional step) of the values of the values matrix, to values that are between 0 and 1, into a matrix of normalized values.

Figure 3 represents a matrix of processed values obtained after processing the matrix of measured data, using the LMaFit algorithm according to the present invention, in which the positions of the parameters detected as erroneous (in dark gray) and/or missing (in light gray and dashes (-)) are highlighted, which are detected in the matrix of normalized values when screening the matrix of processed values.

Figure 4 shows the matrix of screened values resulting from screening the matrix of processed data (optional step), in which said missing values (in light gray, and with a dash (-) previously) or erroneous values (in dark gray), are screened from the matrix by replacing them with null values 0.

Figure 5 shows the matrix of reprocessed values resulting from processing the matrix of screened values using the LMaFit algorithm according to the present invention, in which the null (0) values initially missing (in light gray) or erroneous (in dark gray) have been completed by new values using said algorithm.

## Detailed description of the invention

The object of the present invention is to provide a method for determining water quality. Specifically, after measuring the water quality parameters, these values are grouped into a matrix of measured values to which a mathematical method of matrix completion is applied with a series of conditions already briefly explained, which will be explained in more detail later. This method can be carried out for example using data completion algorithms, such as matrix rank minimization algorithms, or matrix nuclear norm minimization algorithms. In said matrix, as a non-limiting example, the rows correspond to each measurement made of a water sample (at different time points), while the columns refer to the type of water quality parameters measured; or vice versa.

The methodology of the present invention comprises measuring the quality parameters of a plurality of water samples and obtaining them in the form of a matrix of measured values that probably contain erroneous and/or missing parameters, which will be subsequently processed using a matrix completion/reconstruction method, which allows these values to be recovered from the matrix, and thus they can be used for a better estimation and prediction of water quality.

Specifically, the measured parameters are collected in a matrix of measured values, which will later be completed, minimizing the difference between the matrix of processed values and the matrix of measured values, with the condition that the value of the rank of the matrix of processed values to be obtained is equal to or approximates a predetermined value less than the rank of the matrix of measured values, from a finite subset of initially known measured values. This matrix of processed values will preferably be a low rank matrix, for example, of rank 2 or 3.

The matrix of measured values will have a rank equal to or less than the total number of water quality parameters or number of measured water samples (columns or rows) contained in the matrix, therefore, the rank of the matrix of processed values (when minimized according to the present invention) will have a rank less than said total number of water quality parameters or number of measured water samples.

The difference between matrices according to the present invention refers to the difference of subtracting the corresponding positions or cells from both matrices, that is, subtracting the same position or cell of the matrix for both matrices to be subtracted; for all cells of both matrices. For example, the difference between the values in row 2 and column 1 of the matrix of measured values and the matrix of processed values to be obtained.

The rank of a matrix is defined as the maximum number of columns or rows that are linearly independent of each other.

A finite subset in this context refers to the number of known water measurement values (parameters), and which is always less than the number of total values in the matrix.

The method of the present invention allows the water quality to be determined more precisely, which will allow better decisions to be made about its use, disposal or purification. It will also make it possible to use a greater amount of water that would have been discarded using conventional methods, since the values of the parameters reconstructed thanks to the methodology of the present invention (which would be erroneous or missing according to the simple methodology of the prior art based on comparison of parameters or their extrapolation) will allow water flows to be regarded as being of sufficient quality, in contrast to conventional methods that would discard such flows as a consequence of missing or erroneous data. The water quality evaluation methodology of the prior art is based on simple comparison or extrapolation of water quality parameters, so it is unable to make proper use of erroneous or missing parameters.

The better prediction of water quality provided by the methodology of the present invention will also be useful for improving decision making in water purification plants (WWTP: wastewater treatment plants) and will help in the sizing of future WWTPs thanks to the ability to store sets of historical parameters (days, weeks, or years) with greater reliability.

## Parameter measurement

The measurement of water quality parameters refers to parameters collected by sensors and/or measured in laboratory samples for each of the flows or water samples of interest (for example in a WWTP). These parameters are collected at different times during any period of time.

The measurement of water quality parameters is carried out using any measurement method known in the prior art, for example by sensors or chemical methods, without limiting the invention to these. These parameters are biological and/or chemical parameters that can be collected at any stage (for example in the purification stage of a WWTP). These parameters can be stored in any way, for example in data sheets or computer files (such as an Excel spreadsheet).

Nevertheless, these collected parameters usually contain noise due to the very nature of the processes that are being carried out on the water during the measurement (for example, the different processes of a WWTP such as purification), or due to failures in sensors or measuring devices, or to human errors in the measurement or in the laboratory, or to the non-homogeneity or representativeness of the measured sample. All of this results in errors between the collected parameters.

Noise is understood as random variability in a data sample that degrades the quality of said data. Using historical data (rather than a one-time analytical measure) can help minimize such errors.

Another problem of increasing importance is that of missing parameters that have not been able to be measured by measurement equipment, or are missing due to human errors in obtaining or entering the parameters, or have been lost for any reason.

The methodology of the present invention is capable of solving these problems.

Matrix of measured values

Once the water quality parameters have been measured for the different measurement points of one or more measured water samples (for example collected in a wastewater treatment plant, or WWTP), they are compiled into a matrix of measured values for further processing. Said matrix of measured values will comprise the parameters measured for each of the measurement time points or measured water samples.

Parameters can be incorporated into the matrix in any distribution, although the preferred distribution of the parameters in the matrix will be such that each column represents one of the measured parameters and each row represents a measurement time point (each row may be a time measurement of the same water sample or different water samples) in which said parameters have been measured. An alternative layout would be to represent the parameters in rows and the measurement time points (or measured water samples) in columns.

Such measured values can be obtained in situ (i.e. in real time) or obtained from previously stored measured time series. The greater the number of rows and columns of the matrix, that is, the number of measurements of the water and the number of parameters to be measured of said water, the greater the reliability of the values of the resulting matrix of processed values to be subsequently processed from the matrix of measured values.

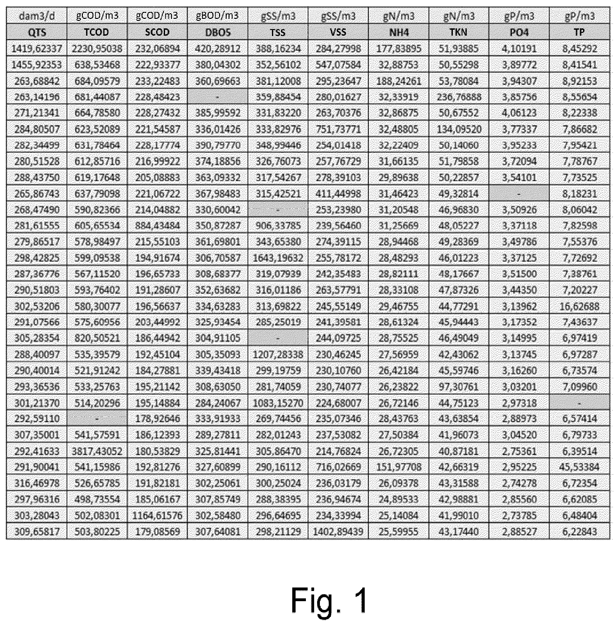

A non-limiting example of a matrix of measured values according to the present invention is represented in Figure 1, showing data collected from a WWTP. The matrix of Fig. 1 consists of 31 rows that represent the measurements made on different water samples taken from a flow for each of the days of a month, and 10 columns that represent the following 10 measured water parameters:

1) The inflow (QTS) with units of cubic decameters per day ($dam^3/d$).
2) Total chemical oxygen demand (TCOD) in grams per cubic meter ($g/m^3$).
3) The soluble chemical oxygen demand (SCOD) in grams per cubic meter ($g/m^3$).
4) Biological oxygen demand (BOD5) in grams per cubic meter ($g/m^3$).
5) Total suspended solids (TSS) in grams per cubic meter ($g/m^3$).
6) Volatile suspended solids (VSS) in grams per cubic meter ($g/m^3$).
7) Ammonium ($NH_4^+$), in grams per cubic meter ($g/m^3$).
8) Total nitrogen (TKN) in grams per cubic meter ($g/m^3$).
9) Phosphate ($PO_4^{-2}$) in grams per cubic meter ($g/m^3$).
10) Total phosphorus (TP) in grams per cubic meter ($g/m^3$)

If there is any modification in the conditions of the water flow between measurements (temperature, changes in the parameters or purification process in a WWTP, etc.), preferably the measured parameters would be measured again under the same conditions, in order not to mix parameters measured in different conditions that could lead to inaccuracies in the subsequent stage of processing or completion of the matrix.

The method of the present invention also comprises measuring water quality parameters for a reference water sample that is already known to be of suitable quality for the use of interest, or the use of previously stored measured parameters of said reference water. The measured parameters of said reference water can be collected in a matrix, which will be referred to as a matrix of reference measured values. The rank value of said matrix of reference measured values can be calculated according to any known method for calculating the rank of a matrix. The usefulness of the value of said rank will be to calibrate the value of the rank of the matrix of processed or reprocessed values to be processed. In other words, to use it as the predetermined value of the rank of the matrix of processed values or reprocessed values to obtain matrices that will be explained below.

**Matrix of processed values**

The matrix of processed values is the matrix resulting from processing the matrix of measured values with the conditions that the value of the rank or nuclear norm of the matrix of processed values is minimized (with respect to the rank or nuclear norm of the matrix of measured values) to a value approximating or equal to a value in the predetermined rank, that is less than the rank of the matrix of measured values, and also that in turn the difference between the resulting processed matrix and the matrix of measured values is minimized.

The rank of a matrix is a concept known in the prior art and can be briefly summarized as *"the number of rows or columns that have the largest invertible submatrix that can be formed"*. The rank of a matrix is also defined as the maximum number of linearly independent rows (or columns). Processes for calculating the rank of a matrix are known in the prior art. Algorithms are also known that are capable of transforming a starting matrix, into a matrix to be obtained of predetermined minor rank, so that the difference between the starting matrix and the obtained matrix is minimized.

Value of the rank approximating a predetermined value of the rank in this specification is understood to mean a value close to said predetermined value, for example of $\pm 1$ or $\pm 2$ with respect to said predetermined rank. As a non-limiting example, if the predetermined value of the rank was 4, the value of the resulting matrix of processed values could be 2, 3, 4, 5 or 6.

When a matrix contains data that are correlated with each other (as is the case with water parameters), that matrix usually has a low rank. However, if noise is added to this data or erroneous data appear, the rank of the matrix increases. Therefore, it would be the erroneous data of the matrix that increases the rank of the matrix, and hence the condition of minimizing the rank of the matrix improves the values of the matrix of processed values obtained. The condition that the difference between the matrix of processed values and the matrix of measured values is minimal also helps to minimize the errors of the processed matrix with respect to the matrix of measured values.

The rank of the matrix of processed values is predetermined so that the obtained matrix has a rank approximating or equal to a predetermined value, rank of the matrix of processed values that will be less than the rank of the matrix of measured values, for example greater than 2 but less than the rank of the matrix of measured values. As a non-limiting example, if the rank of the matrix of measured values was 5, the rank of the matrix of processed values could be for example 2, 3, or 4. Preferably, said rank of the matrix of processed values will be predetermined to rank 2 or rank 3.

In fact, it has been observed that the measured water quality parameters do not have many degrees of freedom, specifically, it has been observed that they tend to be related to 2-3 degrees of freedom (or even less), and therefore the rank of the matrix of measured values are low and constant rank matrices.

This is because water quality parameters describe chemical and/or biological characteristics that are correlated with each other. It is also mainly due to the conditions to which the water is subjected, such as climatic factors or habits of the people who use it, tending to remain relatively constant.

All of this results in a certain homogeneity of water quality parameters over time for a given population, which means that by predetermining the rank of the processed matrix to a value 2 or 3, reconstructed values of the processed matrix are obtained that are accurate or of sufficient quality.

To process the matrix of measured values, any known matrix completion method or algorithm can be used with the conditions set out above, for example algorithms based on the minimization of the rank or the nuclear norm of the matrix (for example OptSpace, SVT, FPCA, etc.).

Furthermore, if the problem is solved with algorithms based on nuclear norm minimization, the particular problem of completeness of water quality parameters becomes convex, and then it can be solved with algorithms based on gradient descent or Lagrank multipliers, which provides advantages in terms of requiring less power and computing time to solve the problem.

To that end, a preferred example of an algorithm to be used (to process the matrix of measured values into a matrix of processed values) would be the algorithm known as LMaFit (disclosed in the article by Zaiwen Wen et al.; DOI: 10.1007/s12532-012-0044-1). This LMaFit algorithm, in the particular case of processing water quality parameters, allows the completion of said missing or erroneous (spurious) parameters of the matrix with lower computational cost, compared to other conventional matrix completion methods or algorithms.

The dimensions of said resulting matrix of processed values consist of each type of measured parameter preferably in columns, measured at different time periods and/or for different water samples preferably in rows; or vice versa.

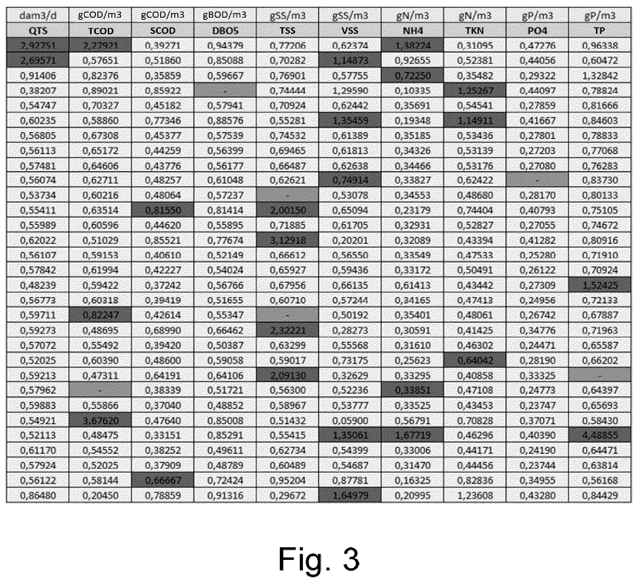

A non-limiting example of a matrix of values processed according to the method of the present invention is shown in Figure 3, using the LMaFit algorithm, in which also after a subsequent screening step, it is possible to detect the positions in which the original values of the matrix of measured values were both missing (detected as a dash (-) and in light gray in Figures 1-3) and erroneous (detected in dark gray in Figure 3).

Although it should be said that Figure 3 is simply illustrative and non-limiting. In fact, the positions (cells) detected with a dash in Figure 3 would have already been completed with a value after processing the matrix of measured values as indicated, but for illustrative purposes, these positions are shown with a dash for easier identification.

Matrix of screened values (optional step)

Screening according to the present invention means discarding or discriminating certain values from the matrix of processed values. The screened values will therefore be the discarded or missing values from the matrix of processed values.

To screen the parameters, missing and/or erroneous values will be identified and discriminated. Those values of the matrix of processed values (that is, cells of said matrix) will be considered erroneous such that the difference between the matrix of processed values and the matrix of measured values (that is, the difference of the value of a cell of the matrix of processed values with respect to the value of the corresponding cell of the matrix of measured values) exceeds a predetermined error or tolerance for each water quality parameter. These corresponding positions of the values of the initial matrix of measured values were in fact the ones that were increasing the rank of the initial matrix of measured values.

This predetermined error can be the same for all water quality parameters, or be different for each water quality parameter (columns). In fact, this predetermined error according to the method can be predetermined based on different criteria, such as the type of water being measured or the acceptable quality margins according to the future use of said water. As a non-limiting example, the predetermined error above which values are screened (discarded) from the matrix of processed values, can be for example greater than or equal to $\pm 50\%$ (of the value of the matrix of processed values with respect to the value of the matrix of measured values), preferably greater than $\pm 30\%$, and even more preferably greater than or equal to $\pm 15\%$. It has been found that the particular value of the tolerance error greater than or equal to 15% (used to detect the values to be screened from the matrix of processed values in Figure 3, highlighted in light gray and dark gray) provides precise identification of those values to be screened that increased the rank of the matrix of measured values and the matrix of processed values before screening, and after screening them and reprocessing the matrix again, discarding said screened values, the precision of the future reprocessed matrix of values to be obtained is increased. After processing, in said positions of the matrix where the values to be screened have been detected, these screened values are not taken into account and are considered as if they were missing.

According to this criterion, the values of the matrix of processed data that will end up being screened into the matrix of screened values, will be the values that increased the rank of the processed matrix of values.

This will allow the matrix of screened values to be reprocessed again into a matrix of reprocessed values, allowing the screened positions of the matrix to be completed if said parameters exceeded a tolerance error and have therefore been rejected, and further increasing the precision of all final parameter values of the matrix of reprocessed values. This step of reprocessing the matrix of screened values (or reprocessed values) will be done with the same conditions that had already been determined previously to process the matrix of values processed from the matrix of measured values, that is, minimizing the difference between the matrix of screened values and the matrix of reprocessed values, and with the condition that the rank of the obtained reprocessed matrix approximates or is equal to a predetermined value less than the rank of the matrix of screened values. With all this, the matrix of reprocessed values will be obtained, which is the one that provides the greatest precision in estimating the water quality according to the invention.

This screening, in a particular and non-limiting manner, can be carried out according to the present invention by means of a matrix completion algorithm that already comprises the application of said screening and reprocessing conditions, such as the algorithm known as LMaFit, which is capable of comparing the matrix of processed values with the matrix of measured values, subtracting both, the LMaFit algorithm that has been used to obtain the screened matrix of Fig. 3. For the example in Fig. 3, a predetermined error greater than or equal to $\pm 15\%$ has been established, which marks the error above which the LMaFit algorithm screens the values of the processed matrix.

Examples of known matrix completion algorithms, that can apply both the processing step, such as optional screening and reprocessing steps, all of them with the conditions defined according to the present invention, are for example OptSpace, SVT, FPCA, etc.; the LMaFit algorithm being the preferred algorithm to be used to perform the processing, as well as optional screening and reprocessing, according to the preferred non-limiting embodiment of the invention.

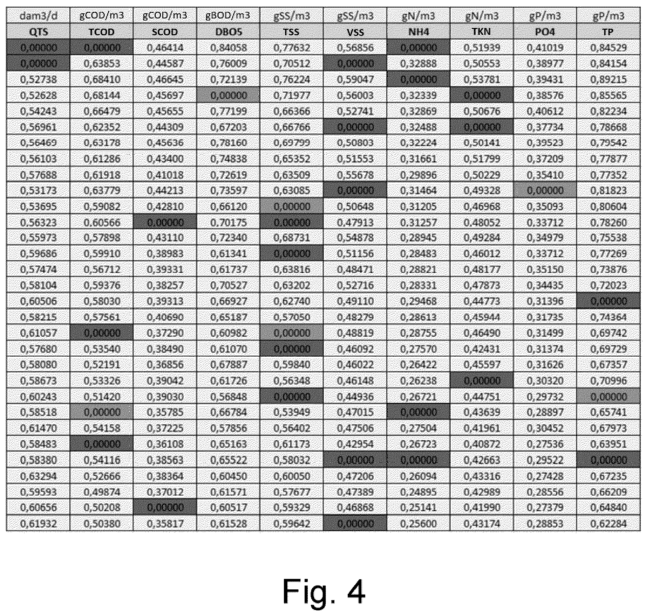

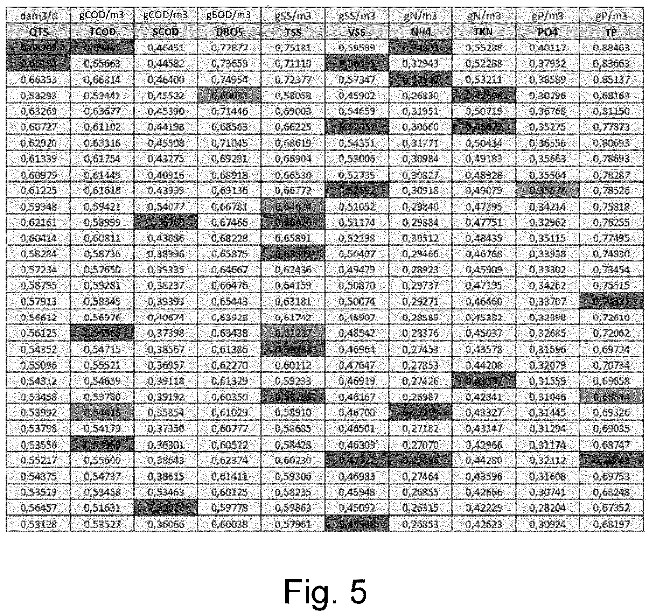

These algorithms therefore manage to reduce the rank of the processed matrix to a value approximating or equal to a value of the predetermined rank. They also allow erroneous values to be screened and reconstructed, maintaining characteristic correlations between water quality parameters. These algorithms are capable of calculating the difference of the matrix of processed values (for example using LMaFit, resulting, for example, in the matrix of Fig. 3) with respect to the matrix of measured values (e.g. Fig. 1), allowing the positions of the values to be screened from the matrix of original measured values to be detected. This will detect erroneous or missing values in the matrix of processed values (e.g. Fig. 3), which will optionally be discarded to obtain the matrix of screened values (e.g. Fig. 4) whose values will be reprocessed according to the present invention to obtain the matrix of reprocessed values (Fig. 5).

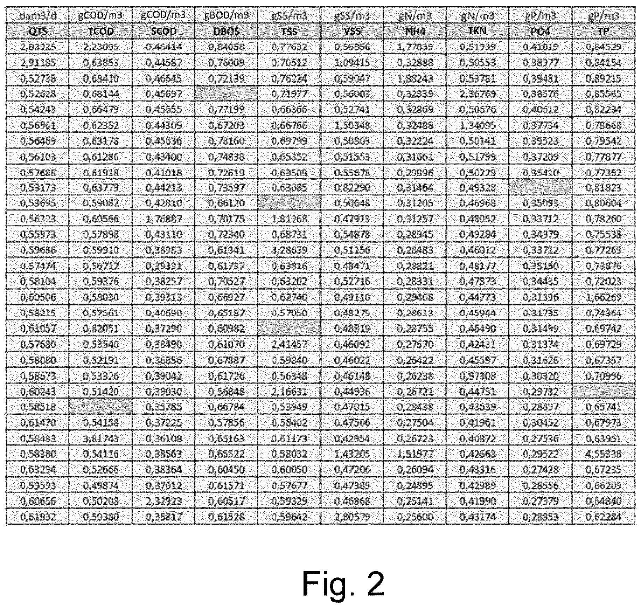

Optionally, before obtaining the matrix of processed data or the matrix of reprocessed data, it is possible to normalize the values collected in the matrix of measured values (or in the matrix of screened values) to normalized values between 0 and 1. Figure 2 shows an example of a normalized matrix of measured values. To that end, the data of each column (values of the same parameter measured in different time measurements of water samples) are divided by the same value that manages to normalize the values of said parameter between 0 and 1, for example, between the maximum value of said parameter for the rest of the time measurements or water measurements. Using matrices with normalized parameters will allow more precise reconstructed values to be obtained after the subsequent processing or reprocessing step, that is, after the reconstruction of the matrix of measured or screened values.

The greater precision of the reconstructed values allows a better determination of water quality and therefore greater efficiency in decisions on reuse, purification or disposal of said water, which will allow a greater amount of water to be reused.

Optionally, normalized values can also be denormalized again at any time, for example, in the final step of determining water quality. To determine the water quality, the values of the matrices of processed or reprocessed values can be compared with acceptance thresholds of said predetermined, or previously known parameters, or those calculated on site. This comparison is more direct when it is done with denormalized values with respect to said parameter value thresholds.

Also optionally, after the screening step, and in order to increase the precision of the subsequent reprocessing step to obtain a matrix of reprocessed values, it can be decided to eliminate time measurements of the water (i.e. the rows of the matrix of screened values) that include a number or percentage of screened values (in Fig. 4 represented with the value 0) equal to or greater than a predetermined maximum number of erroneous or missing values (screened) acceptable for a water measurement. The number of maximum erroneous or missing parameters (columns) to be able to maintain the water measurement (row) in the matrix of screened values is flexible, although it has been observed that the subsequent reprocessing of the matrix of reprocessed values is possible as long as the water measurement (row) contains only 2 or 3 parameters considered acceptable (that is, non-screened, that is, not considered missing or erroneous after screening). A preferred (but non-limiting) and optional criterion for choosing to discard/delete a water measurement (row) is that it contains more than 50% parameters considered erroneous or missing (i.e. screened), preferably more than 90% of parameters considered erroneous or missing, and even more preferably containing less than 3 or 2 parameters considered acceptable (i.e. non-screened).

After carrying out (or not carrying out) this optional step of discarding water samples (rows), the missing or erroneous values of the resulting parameter matrix will be completed using the reprocessing explained above. It has been observed that the presence of at least 2 or 3 acceptable parameters in a row is sufficient to reconstruct it.

Reconstruction of missing or erroneous parameters according to the method of the present invention, specifically from the matrix of measured values in the matrix of processed or reprocessed values (in this case after prior screening), provides the following 3 advantages in the field of water quality estimation:

1) In general, there are typical parameters (or ratios) in order to know what the water quality is like. It is not always possible to determine the water quality if all the parameters to do so are not available. If we reconstruct the values of the erroneous or missing parameters of the matrix (gaps or null values), we will be able to compare with much more reliability the values of these parameters with typical values or ratios of water quality, and thus determine whether the water is of sufficient quality to be able to make a better decision regarding its reuse, purification, or rejection.

2) To carry out mathematical simulations of water treatment, it is necessary to start from the maximum possible analytical measures. From water quality measurements, a simulation model can be built, transforming these measurements into components of the simulation model. In fact, simulation models benefit from screened and reconstructed data for more precise simulation, and furthermore the simulation would not be possible with missing data without reconstructing, a problem that the method of the present invention solves. This allows the creation or improvement of mathematical simulation models for water quality evaluation and/or water treatment, in order to increase efficiency in water use, and optimize and calibrate WWTPs (wastewater treatment plants).

3) If there is a certain level of error in the measurement of the parameters (for example due to lack of precision of the sensors that measure water quality parameters), this could change the correlations between the parameters, increasing the rank of the matrix and thus the errors in determining water quality. Nevertheless, by means of the reconstruction method of the present invention, it would be possible to correct all the values of the parameters of the matrix, mitigating errors in data measurement.

The processing and reprocessing therefore manage to estimate the missing data in the matrix of measured values, and correct any erroneous data included therein, analyzing the set of values of the matrix together, thus being able to complete or reconstruct any value (cell) of the matrix, minimizing its errors.

### LMaFIT algorithm

The operation of this "LMaFit" algorithm is known in the prior art, and it is described in detail, for example, in the following scientific article: Math. Prog. Comp (2012) 4:333-361, by Zaiwen Wen et al. (DOI: 0.1007/s12532-012-0044-1). The operation of the "LMaFit" algorithm and its advantages are explained, briefly below:
The "LMaFit" algorithm is a low-rank model based on the theory presented in the previous article (Zaiwen Wen et al.). In this article, the aim is to solve the following optimization problem expressed mathematically:

$$\min \quad \frac{1}{2}\|XY - Z\|_F^2 \quad \text{s.t.} \quad Z_{ij} = M_{ij}, \quad \forall(i,j) \in \Omega.$$

This problem refers to minimizing, for each iteration, the square of the Frobenius norm (https://mathworld. wolfram.com/FrobeniusNorm.html), subject to (s.t.) the condition that the values estimated by $Z_{i,j}$ are as close as possible to the values of $M_{i,j}$, according to the known positions (i,j).

Other algorithms different to existing LMaFit algorithms are based on singular value decomposition of the matrix to be studied. The foundation of this technique lies in factoring a matrix (e.g. A) into a product of three matrices, (for example 3 matrices U, S, V; with which $A = USV^T$).

According to the internal criteria of said algorithms different to LMaFit, this product of matrices is updated in each iteration in such a way that it meets a tolerance (maximum error allowed) between the values of the original matrix A (matrix of measured values) and the values of the recovered matrix A (matrix of reconstructed values). One of the main problems of this methodology is the increasing computational cost as the matrix to be studied grows in size. This means, the larger the dimensions of A, the greater the computational cost of its singular value decomposition. To that end, the computational cost is very high for these different conventional matrix rank minimization algorithms, such as OptSpace, SVT, FPCA, etc.

On the contrary, the "LMaFit" algorithm preferred in the present invention is based on factoring a matrix M into a product of two matrices, X and Y, using "QR decomposition" (https://mathworld.wolfram.com/QRDecomposition.html) to solve a "least squares problem" (https://textbooks.math.gatech.edu/ila/least-squares.html) that arises in each iteration of the problem, which reduces the computational cost to process large matrices of measured or screened values.

Furthermore, for the particular problem of recovering characteristic parameters of water quality, there is a considerable advantage since the estimated rank of the matrix of measured values is known, and this saves a lot of computing time for the algorithm.

Compared to the use of other reconstruction algorithms in the art (such as "singular value decomposition") the use of the LMaFit algorithm to solve this particular problem of screening and subsequent reconstruction of water quality parameters is faster and more accurate, for this particular reconstruction of water quality parameters.

## Data processing or reprocessing (after screening), using LMaFit

Figures 4-5 according to the present invention show a non-limiting example of the use of the LMaFit algorithm for the data reprocessing step to reconstruct a previously screened matrix ($M_{mini}$). To that end, once the matrix has been screened, screened values are treated as missing values. For example, visually, a null value (0) can be assigned to the positions/cells screened with erroneous data from the matrix, to visually highlight what is considered missing (see Fig. 4). Subsequently, the data is ordered in the form of a vector. In other words, the matrix of screened values in Figure 4 which consists of *mi* rows (31 rows corresponding to daily water measurements for a month) and *ni* columns (10 columns corresponding to 10 water quality parameters) would have dimensions of 310 rows and 1 column in vector form.

By its definition, the LMaFit algorithm needs the following information as a starting point:

> *mi* - number of rows of the screened matrix $Mmi{\times}ni$; for example *mi* = 31.
> *ni* - number of columns of the screened matrix $Mmi{\times}ni$; for example *ni* = 10.
> *k* - estimated rank of the recovered matrix; for example k = 2.
> *x* - known data (i.e. not null) of the screened matrix.
> p - positions "p" of the known data "x", in the data vector.

In this particular example, the estimated rank is predetermined to k = 2 because, for the specific problem being treated, this would demonstrate that the data matrix could be reliably reconstructed from only two of its columns, that is, from 2 types of water quality parameters.

Once the known data has been processed, two matrices are obtained $xmi{\times}k$ and $Yk{\times}ni$, whose matrix product results in the matrix of reprocessed values in Figure 5 ($\tilde{M}mi{\times}ni$); this is $Xmi{\times}k * Yk{\times}ni = \tilde{M}mi{\times}ni$.

The processing step of the matrix of measured values into the matrix of processed values would be done in the same way, with the difference that it would start from the matrix of measured values (for example that of Figure 1) which would be organized as a vector to obtain the matrix of processed values (Figure 2).

## Determination of water quality

Once the parameter values are reconstructed in the matrix of processed or reprocessed values, these values will be compared with predetermined acceptance thresholds for the water quality parameters (or ratios of parameters), in order to determine whether the quality of each of the water samples is sufficient or not for the intended use of said water.

Thus, a decision can be made about what to do with that water, either reuse it, purify it, reject it, etc. These predetermined threshold values that determine whether the water is of sufficient quality (with which it is compared) can either be determined experimentally or be predetermined values in the literature, that is, said predetermined values can be known or estimated in situ.

In summary, the present invention allows optimizing the reuse or purification of water flows, much more precisely and exhaustively than conventional water quality control methods that end up rejecting water flows of sufficient quality. This problem is solved by the method of the present invention, which comprises the measurement of representative parameters of water quality for the different water flows to be evaluated, and their subsequent reconstruction, optionally screening out erroneous and/or missing values and reconstructing them again to increase the precision of the water estimation. This method is capable of correlating water quality parameters that are inherently related to each other, in order to make better decisions to accept or reject water flows.

This contrasts with conventional methods of simply evaluating water quality based on whether any of its parameters fall outside a predetermined rank or threshold that defines the water as being of acceptable quality, where decisions may be based on erroneous or missing data, and/or data that does not correlate, which entails making less precise decisions to reject or accept water. In fact, conventional water treatments and analyzes focus on characterizing the water and analyzing the characterized variables (e.g. chemical oxygen demand, ammonium, phosphate, etc.) individually.

In the present invention we have gone further, since it has been observed that these characteristic variables of water quality are interrelated with each other, and said correlation that the inventor has identified has been used to organize the measured water quality parameters in a matrix (matrix of measured values), and thus be able to reconstruct these matrices, optionally screening for erroneous and/or missing parameters, with the advantages previously stated.

Furthermore, the parameters obtained by the method of the present invention will also allow the construction of better water quality simulation models, for example to optimize and calibrate WWTPs (wastewater treatment plants).

Lastly, it should be noted that only some embodiments of the invention are described herein, therefore, the person skilled in the art will understand that other equivalent or alternative embodiments of the invention are also possible, as well as its obvious and equivalent modifications. For this reason, the scope of the aspects of the invention should not be limited to the specific embodiments specifically described.

**Claims**

1. A method for determining whether water quality is suitable for subsequent use, comprising the steps of:

   - measuring a plurality of quality parameters of different water samples at different time points, obtaining a matrix of measured values of parameters for the different time points;
   - processing the matrix of measured values to obtain a matrix of processed values such that the difference between said matrices is minimal with the condition that the value of the rank of the matrix of processed values approximates or is equal to a predetermined value less than the rank of the matrix of measured values, the rank of the matrix of processed values preferably being equal to 2 or 3;
   - determining water quality by comparing values from the matrix of processed values with predetermined acceptance thresholds for water quality parameters.

2. The method for determining whether water quality is suitable for subsequent use, according to claim 1, comprising:

   - screening the matrix of processed values, screening the values of the matrix such that the difference between the processed values and the measured values, or said difference with respect to the corresponding measured values, exceeds a predetermined error;
   - reprocessing the matrix of screened values to obtain a matrix of reprocessed values such that the difference between said matrices is minimal with the condition that the value of the rank of the matrix of reprocessed values approximates or is equal to a predetermined value less than the rank of the matrix of screened values, the rank of the matrix of processed values preferably being equal to 2 or 3; and
   - determining water quality by comparing values from the matrix of reprocessed values, instead of the matrix of processed values, with predetermined acceptance thresholds for water quality parameters.

3. The method according to any of claims 1 or 2, in which to process the matrix of measured values or the matrix of screened values, a matrix completion algorithm is used, which is an algorithm based on the minimization of the rank or nuclear norm of the matrix.

4. The method according to any of claims 1 to 3, such that the predetermined value of the rank of the matrix of processed or reprocessed values is determined from the rank of a matrix of reference measured values, obtained for a reference water sample measured at different time points.

5. The method according to any of claims 2 to 4, in which in the matrix of screened values, those measurements of water samples are eliminated, whose measured values contain a number of screened values greater than 50% of the number of total parameters of the water sample measurement, preferably greater than 90%, or those measurements of water samples that do not have at least 2 non-screened values are eliminated.

6. The method according to any of claims 1 to 5, in which prior to processing the matrix of measured values or reprocessing the matrix of screened values, the values of the matrix are normalized to values that are between 0 and 1.

7. The method for determining whether water quality is suitable according to any of claims 1 to 6, which is used for wastewater treatment.

| dam3/d | gCOD/m3 | gCOD/m3 | gBOD/m3 | gSS/m3 | gSS/m3 | gN/m3 | gN/m3 | gP/m3 | gP/m3 |
|---|---|---|---|---|---|---|---|---|---|
| QTS | TCOD | SCOD | DBOS | TSS | VSS | NH4 | TKN | PO4 | TP |
| 1419,62337 | 2230,95038 | 232,06894 | 420,28912 | 388,16234 | 284,27998 | 177,83895 | 51,93885 | 4,10191 | 8,45292 |
| 1455,92353 | 638,53468 | 222,93377 | 380,04302 | 352,56102 | 547,07584 | 32,88753 | 50,55298 | 3,89772 | 8,41541 |
| 263,68842 | 684,09579 | 233,22483 | 360,69663 | 381,12008 | 295,23647 | 188,24261 | 53,78084 | 3,94307 | 8,92153 |
| 263,14196 | 681,44087 | 228,48423 | - | 359,88454 | 280,01627 | 32,33919 | 236,76888 | 3,85756 | 8,55654 |
| 271,21341 | 664,78580 | 228,27432 | 385,99592 | 331,83220 | 263,70376 | 32,86875 | 50,67552 | 4,06123 | 8,22338 |
| 284,80507 | 623,52089 | 221,54587 | 336,01426 | 333,82976 | 751,73771 | 32,48805 | 134,09520 | 3,77337 | 7,86682 |
| 282,34499 | 631,78464 | 228,17774 | 390,79770 | 348,99446 | 254,01418 | 32,22409 | 50,14060 | 3,95233 | 7,95421 |
| 280,51528 | 612,85716 | 216,99922 | 374,18856 | 326,76073 | 257,76729 | 31,66135 | 51,79858 | 3,72094 | 7,78767 |
| 288,43750 | 619,17648 | 205,08883 | 363,09332 | 317,54267 | 278,39103 | 29,89638 | 50,22857 | 3,54101 | 7,73525 |
| 265,86743 | 637,79098 | 221,06722 | 367,98483 | 315,42521 | 411,44998 | 31,46423 | 49,32814 | - | 8,18231 |
| 268,47490 | 590,82366 | 214,04882 | 330,60042 | - | 253,23980 | 31,20548 | 46,96830 | 3,50926 | 8,06042 |
| 281,61555 | 605,65534 | 884,43484 | 350,87287 | 906,33785 | 239,56460 | 31,25669 | 48,05227 | 3,37118 | 7,82598 |
| 279,86517 | 578,98497 | 215,55103 | 361,69801 | 343,65380 | 274,39115 | 28,94468 | 49,28369 | 3,49786 | 7,55376 |
| 298,42825 | 599,09538 | 194,91674 | 306,70587 | 1643,19632 | 255,78172 | 28,48293 | 46,01223 | 3,37125 | 7,72692 |
| 287,36776 | 567,11520 | 196,65733 | 308,68377 | 319,07939 | 242,35483 | 28,82111 | 48,17667 | 3,51500 | 7,38761 |
| 290,51803 | 593,76402 | 191,28607 | 352,63682 | 316,01186 | 263,57791 | 28,33108 | 47,87326 | 3,44350 | 7,20227 |
| 302,53206 | 580,30077 | 196,56637 | 334,63283 | 313,69822 | 245,55149 | 29,46755 | 44,77291 | 3,13962 | 16,62688 |
| 291,07566 | 575,60956 | 203,44992 | 325,93454 | 285,25019 | 241,39581 | 28,61324 | 45,94443 | 3,17352 | 7,43637 |
| 305,28354 | 820,50521 | 186,44942 | 304,91105 | - | 244,09725 | 28,75525 | 46,49049 | 3,14995 | 6,97419 |
| 288,40097 | 535,39579 | 192,45104 | 305,35093 | 1207,28338 | 230,46245 | 27,56959 | 42,43062 | 3,13745 | 6,97287 |
| 290,40014 | 521,91242 | 184,27881 | 339,43418 | 299,19759 | 230,10760 | 26,42184 | 45,59746 | 3,16260 | 6,73574 |
| 293,36536 | 533,25763 | 195,21142 | 308,63050 | 281,74059 | 230,74077 | 26,23822 | 97,30761 | 3,03201 | 7,09960 |
| 301,21370 | 514,20296 | 195,14884 | 284,24067 | 1083,15270 | 224,68007 | 26,72146 | 44,75123 | 2,97318 | - |
| 292,59110 | - | 178,92646 | 333,91933 | 269,74456 | 235,07346 | 28,43763 | 43,63854 | 2,88973 | 6,57414 |
| 307,35001 | 541,57591 | 186,12393 | 289,27811 | 282,01243 | 237,53082 | 27,50384 | 41,96073 | 3,04520 | 6,79733 |
| 292,41633 | 3817,43052 | 180,53829 | 325,81441 | 305,86470 | 214,76824 | 26,72305 | 40,87181 | 2,75361 | 6,39514 |
| 291,90041 | 541,15986 | 192,81276 | 327,60899 | 290,16112 | 716,02669 | 151,97708 | 42,66319 | 2,95225 | 45,53384 |
| 316,46978 | 526,65785 | 191,82181 | 302,25061 | 300,25024 | 236,03179 | 26,09378 | 43,31588 | 2,74278 | 6,72354 |
| 297,96316 | 498,73554 | 185,06167 | 307,85749 | 288,38395 | 236,94674 | 24,89533 | 42,98881 | 2,85560 | 6,62085 |
| 303,28043 | 502,08301 | 1164,61576 | 302,58480 | 296,64695 | 234,33994 | 25,14084 | 41,99010 | 2,73785 | 6,48404 |
| 309,65817 | 503,80225 | 179,08569 | 307,64081 | 298,21129 | 1402,89439 | 25,59955 | 43,17440 | 2,88527 | 6,22843 |

## Fig. 1

| dam3/d | gCOD/m3 | gCOD/m3 | gBOD/m3 | gSS/m3 | gSS/m3 | gN/m3 | gN/m3 | gP/m3 | gP/m3 |
|--------|---------|---------|---------|--------|--------|-------|-------|-------|-------|
| QTS | TCOD | SCOD | DBO5 | TSS | VSS | NH4 | TKN | PO4 | TP |
| 2,83925 | 2,23095 | 0,46414 | 0,84058 | 0,77632 | 0,56856 | 1,77839 | 0,51939 | 0,41019 | 0,84529 |
| 2,91185 | 0,63853 | 0,44587 | 0,76009 | 0,70512 | 1,09415 | 0,32888 | 0,50553 | 0,38977 | 0,84154 |
| 0,52738 | 0,68410 | 0,46645 | 0,72139 | 0,76224 | 0,59047 | 1,88243 | 0,53781 | 0,39431 | 0,89215 |
| 0,52628 | 0,68144 | 0,45697 | - | 0,71977 | 0,56003 | 0,32339 | 2,36769 | 0,38576 | 0,85565 |
| 0,54243 | 0,66479 | 0,45655 | 0,77199 | 0,66366 | 0,52741 | 0,32869 | 0,50676 | 0,40612 | 0,82234 |
| 0,56961 | 0,62352 | 0,44309 | 0,67203 | 0,66766 | 1,50348 | 0,32488 | 1,34095 | 0,37734 | 0,78668 |
| 0,56469 | 0,63178 | 0,45636 | 0,78160 | 0,69799 | 0,50803 | 0,32224 | 0,50141 | 0,39523 | 0,79542 |
| 0,56103 | 0,61286 | 0,43400 | 0,74838 | 0,65352 | 0,51553 | 0,31661 | 0,51799 | 0,37209 | 0,77877 |
| 0,57688 | 0,61918 | 0,41018 | 0,72619 | 0,63509 | 0,55678 | 0,29896 | 0,50229 | 0,35410 | 0,77352 |
| 0,53173 | 0,63779 | 0,44213 | 0,73597 | 0,63085 | 0,82290 | 0,31464 | 0,49328 | - | 0,81823 |
| 0,53695 | 0,59082 | 0,42810 | 0,66120 | - | 0,50648 | 0,31205 | 0,46968 | 0,35093 | 0,80604 |
| 0,56323 | 0,60566 | 1,76887 | 0,70175 | 1,81268 | 0,47913 | 0,31257 | 0,48052 | 0,33712 | 0,78260 |
| 0,55973 | 0,57898 | 0,43110 | 0,72340 | 0,68731 | 0,54878 | 0,28945 | 0,49284 | 0,34979 | 0,75538 |
| 0,59686 | 0,59910 | 0,38983 | 0,61341 | 3,28639 | 0,51156 | 0,28483 | 0,46012 | 0,33712 | 0,77269 |
| 0,57474 | 0,56712 | 0,39331 | 0,61737 | 0,63816 | 0,48471 | 0,28821 | 0,48177 | 0,35150 | 0,73876 |
| 0,58104 | 0,59376 | 0,38257 | 0,70527 | 0,63202 | 0,52716 | 0,28331 | 0,47873 | 0,34435 | 0,72023 |
| 0,60506 | 0,58030 | 0,39313 | 0,66927 | 0,62740 | 0,49110 | 0,29468 | 0,44773 | 0,31396 | 1,66269 |
| 0,58215 | 0,57561 | 0,40690 | 0,65187 | 0,57050 | 0,48279 | 0,28613 | 0,45944 | 0,31735 | 0,74364 |
| 0,61057 | 0,82051 | 0,37290 | 0,60982 | - | 0,48819 | 0,28755 | 0,46490 | 0,31499 | 0,69742 |
| 0,57680 | 0,53540 | 0,38490 | 0,61070 | 2,41457 | 0,46092 | 0,27570 | 0,42431 | 0,31374 | 0,69729 |
| 0,58080 | 0,52191 | 0,36856 | 0,67887 | 0,59840 | 0,46022 | 0,26422 | 0,45597 | 0,31626 | 0,67357 |
| 0,58673 | 0,53326 | 0,39042 | 0,61726 | 0,56348 | 0,46148 | 0,26238 | 0,97308 | 0,30320 | 0,70996 |
| 0,60243 | 0,51420 | 0,39030 | 0,56848 | 2,16631 | 0,44936 | 0,26721 | 0,44751 | 0,29732 | - |
| 0,58518 | - | 0,35785 | 0,66784 | 0,53949 | 0,47015 | 0,28438 | 0,43639 | 0,28897 | 0,65741 |
| 0,61470 | 0,54158 | 0,37225 | 0,57856 | 0,56402 | 0,47506 | 0,27504 | 0,41961 | 0,30452 | 0,67973 |
| 0,58483 | 3,81743 | 0,36108 | 0,65163 | 0,61173 | 0,42954 | 0,26723 | 0,40872 | 0,27536 | 0,63951 |
| 0,58380 | 0,54116 | 0,38563 | 0,65522 | 0,58032 | 1,43205 | 1,51977 | 0,42663 | 0,29522 | 4,55338 |
| 0,63294 | 0,52666 | 0,38364 | 0,60450 | 0,60050 | 0,47206 | 0,26094 | 0,43316 | 0,27428 | 0,67235 |
| 0,59593 | 0,49874 | 0,37012 | 0,61571 | 0,57677 | 0,47389 | 0,24895 | 0,42989 | 0,28556 | 0,66209 |
| 0,60656 | 0,50208 | 2,32923 | 0,60517 | 0,59329 | 0,46868 | 0,25141 | 0,41990 | 0,27379 | 0,64840 |
| 0,61932 | 0,50380 | 0,35817 | 0,61528 | 0,59642 | 2,80579 | 0,25600 | 0,43174 | 0,28853 | 0,62284 |

# Fig. 2

| dam3/d | gCOD/m3 | gCOD/m3 | gBOD/m3 | gSS/m3 | gSS/m3 | gN/m3 | gN/m3 | gP/m3 | gP/m3 |
|---|---|---|---|---|---|---|---|---|---|
| QTS | TCOD | SCOD | DBO5 | TSS | VSS | NH4 | TKN | PO4 | TP |
| 2,92751 | 2,27921 | 0,39271 | 0,94379 | 0,77206 | 0,62374 | 1,38224 | 0,31095 | 0,47276 | 0,96338 |
| 2,69571 | 0,57651 | 0,51860 | 0,85088 | 0,70282 | 1,14873 | 0,92655 | 0,52381 | 0,44056 | 0,60472 |
| 0,91406 | 0,82376 | 0,35859 | 0,59667 | 0,76901 | 0,57755 | 0,72250 | 0,35482 | 0,29322 | 1,32842 |
| 0,38207 | 0,89021 | 0,85922 | - | 0,74444 | 1,29590 | 0,10335 | 1,25267 | 0,44097 | 0,78824 |
| 0,54747 | 0,70327 | 0,45182 | 0,57941 | 0,70924 | 0,62442 | 0,35691 | 0,54541 | 0,27859 | 0,81666 |
| 0,60235 | 0,58860 | 0,77346 | 0,88576 | 0,55281 | 1,35459 | 0,19348 | 1,14911 | 0,41667 | 0,84603 |
| 0,56805 | 0,67308 | 0,45377 | 0,57539 | 0,74532 | 0,61389 | 0,35185 | 0,53436 | 0,27801 | 0,78833 |
| 0,56113 | 0,65172 | 0,44259 | 0,56399 | 0,69465 | 0,61813 | 0,34326 | 0,53139 | 0,27203 | 0,77068 |
| 0,57481 | 0,64606 | 0,43776 | 0,56177 | 0,66487 | 0,62638 | 0,34466 | 0,53176 | 0,27080 | 0,76283 |
| 0,56074 | 0,62711 | 0,48257 | 0,61048 | 0,62621 | 0,74914 | 0,33827 | 0,62422 | - | 0,83730 |
| 0,53734 | 0,60216 | 0,48064 | 0,57237 | - | 0,53078 | 0,34553 | 0,48680 | 0,28170 | 0,80133 |
| 0,55411 | 0,63514 | 0,81550 | 0,81414 | 2,00150 | 0,65094 | 0,23179 | 0,74404 | 0,40793 | 0,75105 |
| 0,55989 | 0,60596 | 0,44620 | 0,55895 | 0,71885 | 0,61705 | 0,32931 | 0,52827 | 0,27055 | 0,74672 |
| 0,62022 | 0,51029 | 0,85521 | 0,77674 | 3,12918 | 0,20201 | 0,32089 | 0,43394 | 0,41282 | 0,80916 |
| 0,56107 | 0,59153 | 0,40610 | 0,52149 | 0,66612 | 0,56550 | 0,33549 | 0,47533 | 0,25280 | 0,71910 |
| 0,57842 | 0,61994 | 0,42227 | 0,54024 | 0,65927 | 0,59436 | 0,33172 | 0,50491 | 0,26122 | 0,70924 |
| 0,48239 | 0,59422 | 0,37242 | 0,56766 | 0,67956 | 0,66135 | 0,61413 | 0,43442 | 0,27309 | 1,52425 |
| 0,56773 | 0,60318 | 0,39419 | 0,51655 | 0,60710 | 0,57244 | 0,34165 | 0,47413 | 0,24956 | 0,72133 |
| 0,59711 | 0,82247 | 0,42614 | 0,55347 | - | 0,50192 | 0,35401 | 0,48061 | 0,26742 | 0,67887 |
| 0,59273 | 0,48695 | 0,68990 | 0,66462 | 2,32221 | 0,28273 | 0,30591 | 0,41425 | 0,34776 | 0,71963 |
| 0,57072 | 0,55492 | 0,39420 | 0,50387 | 0,63299 | 0,55568 | 0,31610 | 0,46302 | 0,24471 | 0,65587 |
| 0,52025 | 0,60390 | 0,48600 | 0,59058 | 0,59017 | 0,73175 | 0,25623 | 0,64042 | 0,28190 | 0,66202 |
| 0,59213 | 0,47311 | 0,64191 | 0,64106 | 2,09130 | 0,32629 | 0,33295 | 0,40858 | 0,33325 | - |
| 0,57962 | - | 0,38339 | 0,51721 | 0,56300 | 0,52236 | 0,33851 | 0,47108 | 0,24773 | 0,64397 |
| 0,59883 | 0,55866 | 0,37040 | 0,48852 | 0,58967 | 0,53777 | 0,33525 | 0,43453 | 0,23747 | 0,65693 |
| 0,54921 | 3,67620 | 0,47640 | 0,85008 | 0,51432 | 0,05900 | 0,56791 | 0,70828 | 0,37071 | 0,58430 |
| 0,52113 | 0,48475 | 0,33151 | 0,85291 | 0,55415 | 1,35061 | 1,67719 | 0,46296 | 0,40390 | 4,48855 |
| 0,61170 | 0,54552 | 0,38252 | 0,49611 | 0,62734 | 0,54399 | 0,33006 | 0,44171 | 0,24190 | 0,64471 |
| 0,57924 | 0,52025 | 0,37909 | 0,48789 | 0,60489 | 0,54687 | 0,31470 | 0,44456 | 0,23744 | 0,63814 |
| 0,56122 | 0,58144 | 0,66667 | 0,72424 | 0,95204 | 0,87781 | 0,16325 | 0,82836 | 0,34955 | 0,56168 |
| 0,86480 | 0,20450 | 0,78859 | 0,91316 | 0,29672 | 1,64979 | 0,20995 | 1,23608 | 0,43280 | 0,84429 |

# Fig. 3

| dam3/d | gCOD/m3 | gCOD/m3 | gBOD/m3 | gSS/m3 | gSS/m3 | gN/m3 | gN/m3 | gP/m3 | gP/m3 |
|---|---|---|---|---|---|---|---|---|---|
| QTS | TCOD | SCOD | DBO5 | TSS | VSS | NH4 | TKN | PO4 | TP |
| 0,00000 | 0,00000 | 0,46414 | 0,84058 | 0,77632 | 0,56856 | 0,00000 | 0,51939 | 0,41019 | 0,84529 |
| 0,00000 | 0,63853 | 0,44587 | 0,76009 | 0,70512 | 0,00000 | 0,32888 | 0,50553 | 0,38977 | 0,84154 |
| 0,52738 | 0,68410 | 0,46645 | 0,72139 | 0,76224 | 0,59047 | 0,00000 | 0,53781 | 0,39431 | 0,89215 |
| 0,52628 | 0,68144 | 0,45697 | 0,00000 | 0,71977 | 0,56003 | 0,32339 | 0,00000 | 0,38576 | 0,85565 |
| 0,54243 | 0,66479 | 0,45655 | 0,77199 | 0,66366 | 0,52741 | 0,32869 | 0,50676 | 0,40612 | 0,82234 |
| 0,56961 | 0,62352 | 0,44309 | 0,67203 | 0,66766 | 0,00000 | 0,32488 | 0,00000 | 0,37734 | 0,78668 |
| 0,56469 | 0,63178 | 0,45636 | 0,78160 | 0,69799 | 0,50803 | 0,32224 | 0,50141 | 0,39523 | 0,79542 |
| 0,56103 | 0,61286 | 0,43400 | 0,74838 | 0,65352 | 0,51553 | 0,31661 | 0,51799 | 0,37209 | 0,77877 |
| 0,57688 | 0,61918 | 0,41018 | 0,72619 | 0,63509 | 0,55678 | 0,29896 | 0,50229 | 0,35410 | 0,77352 |
| 0,53173 | 0,63779 | 0,44213 | 0,73597 | 0,63085 | 0,00000 | 0,31464 | 0,49328 | 0,00000 | 0,81823 |
| 0,53695 | 0,59082 | 0,42810 | 0,66120 | 0,00000 | 0,50648 | 0,31205 | 0,46968 | 0,35093 | 0,80604 |
| 0,56323 | 0,60566 | 0,00000 | 0,70175 | 0,00000 | 0,47913 | 0,31257 | 0,48052 | 0,33712 | 0,78260 |
| 0,55973 | 0,57898 | 0,43110 | 0,72340 | 0,68731 | 0,54878 | 0,28945 | 0,49284 | 0,34979 | 0,75538 |
| 0,59686 | 0,59910 | 0,38983 | 0,61341 | 0,00000 | 0,51156 | 0,28483 | 0,46012 | 0,33712 | 0,77269 |
| 0,57474 | 0,56712 | 0,39331 | 0,61737 | 0,63816 | 0,48471 | 0,28821 | 0,48177 | 0,35150 | 0,73876 |
| 0,58104 | 0,59376 | 0,38257 | 0,70527 | 0,63202 | 0,52716 | 0,28331 | 0,47873 | 0,34435 | 0,72023 |
| 0,60506 | 0,58030 | 0,39313 | 0,66927 | 0,62740 | 0,49110 | 0,29468 | 0,44773 | 0,31396 | 0,00000 |
| 0,58215 | 0,57561 | 0,40690 | 0,65187 | 0,57050 | 0,48279 | 0,28613 | 0,45944 | 0,31735 | 0,74364 |
| 0,61057 | 0,00000 | 0,37290 | 0,60982 | 0,00000 | 0,48819 | 0,28755 | 0,46490 | 0,31499 | 0,69742 |
| 0,57680 | 0,53540 | 0,38490 | 0,61070 | 0,00000 | 0,46092 | 0,27570 | 0,42431 | 0,31374 | 0,69729 |
| 0,58080 | 0,52191 | 0,36856 | 0,67887 | 0,59840 | 0,46022 | 0,26422 | 0,45597 | 0,31626 | 0,67357 |
| 0,58673 | 0,53326 | 0,39042 | 0,61726 | 0,56348 | 0,46148 | 0,26238 | 0,00000 | 0,30320 | 0,70996 |
| 0,60243 | 0,51420 | 0,39030 | 0,56848 | 0,00000 | 0,44936 | 0,26721 | 0,44751 | 0,29732 | 0,00000 |
| 0,58518 | 0,00000 | 0,35785 | 0,66784 | 0,53949 | 0,47015 | 0,00000 | 0,43639 | 0,28897 | 0,65741 |
| 0,61470 | 0,54158 | 0,37225 | 0,57856 | 0,56402 | 0,47506 | 0,27504 | 0,41961 | 0,30452 | 0,67973 |
| 0,58483 | 0,00000 | 0,36108 | 0,65163 | 0,61173 | 0,42954 | 0,26723 | 0,40872 | 0,27536 | 0,63951 |
| 0,58380 | 0,54116 | 0,38563 | 0,65522 | 0,58032 | 0,00000 | 0,00000 | 0,42663 | 0,29522 | 0,00000 |
| 0,63294 | 0,52666 | 0,38364 | 0,60450 | 0,60050 | 0,47206 | 0,26094 | 0,43316 | 0,27428 | 0,67235 |
| 0,59593 | 0,49874 | 0,37012 | 0,61571 | 0,57677 | 0,47389 | 0,24895 | 0,42989 | 0,28556 | 0,66209 |
| 0,60656 | 0,50208 | 0,00000 | 0,60517 | 0,59329 | 0,46868 | 0,25141 | 0,41990 | 0,27379 | 0,64840 |
| 0,61932 | 0,50380 | 0,35817 | 0,61528 | 0,59642 | 0,00000 | 0,25600 | 0,43174 | 0,28853 | 0,62284 |

Fig. 4

| dam3/d | gCOD/m3 | gCOD/m3 | gBOD/m3 | gSS/m3 | gSS/m3 | gN/m3 | gN/m3 | gP/m3 | gP/m3 |
|---|---|---|---|---|---|---|---|---|---|
| QTS | TCOD | SCOD | DBO5 | TSS | VSS | NH4 | TKN | PO4 | TP |
| 0,68909 | 0,69435 | 0,46451 | 0,77877 | 0,75181 | 0,59589 | 0,34833 | 0,55288 | 0,40117 | 0,88463 |
| 0,65183 | 0,65663 | 0,44582 | 0,73653 | 0,71110 | 0,56355 | 0,32943 | 0,52288 | 0,37932 | 0,83663 |
| 0,66353 | 0,66814 | 0,46400 | 0,74954 | 0,72377 | 0,57347 | 0,33522 | 0,53211 | 0,38589 | 0,85137 |
| 0,53293 | 0,53441 | 0,45522 | 0,60031 | 0,58058 | 0,45902 | 0,26830 | 0,42608 | 0,30796 | 0,68163 |
| 0,63269 | 0,63677 | 0,45390 | 0,71446 | 0,69003 | 0,54659 | 0,31951 | 0,50719 | 0,36768 | 0,81150 |
| 0,60727 | 0,61102 | 0,44198 | 0,68563 | 0,66225 | 0,52451 | 0,30660 | 0,48672 | 0,35275 | 0,77873 |
| 0,62920 | 0,63316 | 0,45508 | 0,71045 | 0,68619 | 0,54351 | 0,31771 | 0,50434 | 0,36556 | 0,80693 |
| 0,61339 | 0,61754 | 0,43275 | 0,69281 | 0,66904 | 0,53006 | 0,30984 | 0,49183 | 0,35663 | 0,78693 |
| 0,60979 | 0,61449 | 0,40916 | 0,68918 | 0,66530 | 0,52735 | 0,30827 | 0,48928 | 0,35504 | 0,78287 |
| 0,61225 | 0,61618 | 0,43999 | 0,69136 | 0,66772 | 0,52892 | 0,30918 | 0,49079 | 0,35578 | 0,78526 |
| 0,59348 | 0,59421 | 0,54077 | 0,66781 | 0,64624 | 0,51052 | 0,29840 | 0,47395 | 0,34214 | 0,75818 |
| 0,62161 | 0,58999 | 1,76760 | 0,67466 | 0,66620 | 0,51174 | 0,29884 | 0,47751 | 0,32962 | 0,76255 |
| 0,60414 | 0,60811 | 0,43086 | 0,68228 | 0,65891 | 0,52198 | 0,30512 | 0,48435 | 0,35115 | 0,77495 |
| 0,58284 | 0,58736 | 0,38996 | 0,65875 | 0,63591 | 0,50407 | 0,29466 | 0,46768 | 0,33938 | 0,74830 |
| 0,57234 | 0,57650 | 0,39335 | 0,64667 | 0,62436 | 0,49479 | 0,28923 | 0,45909 | 0,33302 | 0,73454 |
| 0,58795 | 0,59281 | 0,38237 | 0,66476 | 0,64159 | 0,50870 | 0,29737 | 0,47195 | 0,34262 | 0,75515 |
| 0,57913 | 0,58345 | 0,39393 | 0,65443 | 0,63181 | 0,50074 | 0,29271 | 0,46460 | 0,33707 | 0,74337 |
| 0,56612 | 0,56976 | 0,40674 | 0,63928 | 0,61742 | 0,48907 | 0,28589 | 0,45382 | 0,32898 | 0,72610 |
| 0,56125 | 0,56565 | 0,37398 | 0,63438 | 0,61237 | 0,48542 | 0,28376 | 0,45037 | 0,32685 | 0,72062 |
| 0,54352 | 0,54715 | 0,38567 | 0,61386 | 0,59282 | 0,46964 | 0,27453 | 0,43578 | 0,31596 | 0,69724 |
| 0,55096 | 0,55521 | 0,36957 | 0,62270 | 0,60112 | 0,47647 | 0,27853 | 0,44208 | 0,32079 | 0,70734 |
| 0,54312 | 0,54659 | 0,39118 | 0,61329 | 0,59233 | 0,46919 | 0,27426 | 0,43537 | 0,31559 | 0,69658 |
| 0,53458 | 0,53780 | 0,39192 | 0,60350 | 0,58295 | 0,46167 | 0,26987 | 0,42841 | 0,31046 | 0,68544 |
| 0,53992 | 0,54418 | 0,35854 | 0,61029 | 0,58910 | 0,46700 | 0,27299 | 0,43327 | 0,31445 | 0,69326 |
| 0,53798 | 0,54179 | 0,37350 | 0,60777 | 0,58685 | 0,46501 | 0,27182 | 0,43147 | 0,31294 | 0,69035 |
| 0,53556 | 0,53959 | 0,36301 | 0,60522 | 0,58428 | 0,46309 | 0,27070 | 0,42966 | 0,31174 | 0,68747 |
| 0,55217 | 0,55600 | 0,38643 | 0,62374 | 0,60230 | 0,47722 | 0,27896 | 0,44280 | 0,32112 | 0,70848 |
| 0,54375 | 0,54737 | 0,38615 | 0,61411 | 0,59306 | 0,46983 | 0,27464 | 0,43596 | 0,31608 | 0,69753 |
| 0,53519 | 0,53458 | 0,53463 | 0,60125 | 0,58235 | 0,45948 | 0,26855 | 0,42666 | 0,30741 | 0,68248 |
| 0,56457 | 0,51631 | 2,33020 | 0,59778 | 0,59863 | 0,45092 | 0,26315 | 0,42229 | 0,28204 | 0,67352 |
| 0,53128 | 0,53527 | 0,36066 | 0,60038 | 0,57961 | 0,45938 | 0,26853 | 0,42623 | 0,30924 | 0,68197 |

# Fig. 5

# INTERNATIONAL SEARCH REPORT

| | International application No. |
|---|---|
| | PCT/ES2022/070777 |

**A. CLASSIFICATION OF SUBJECT MATTER**

*G01N33/18* (2006.01)
*C02F9/00* (2023.01)

According to International Patent Classification (IPC) or to both national classification and IPC

**B. FIELDS SEARCHED**

Minimum documentation searched (classification system followed by classification symbols)
G01N, C02F

Documentation searched other than minimum documentation to the extent that such documents are included in the fields searched

Electronic data base consulted during the international search (name of data base and, where practicable, search terms used)

EPODOC, INVENES, SEARCH TOOL (EPO), ESPACENET, INTERNET, NPL, WPIAP, WPI

**C. DOCUMENTS CONSIDERED TO BE RELEVANT**

| Category* | Citation of document, with indication, where appropriate, of the relevant passages | Relevant to claim No. |
|---|---|---|
| A | CN 110470600 A (CHINA PETROLEUM & CHEM CORP ET AL.) 19/11/2019, claims | 1-7 |
| A | US 2008262739 A1 (WIDDER MARK W ET AL.) 23/10/2008, Abstract | 1-7 |
| A | US 2008011061 A1 (SIHALLA ZAKARIA) 17/01/2008, Abstract | 1-7 |
| A | CN 101573302 A (ENVIRONMENTAL BIOTECHNOLOGY CR) 04/11/2009, Abstract | 1-7 |

☐ Further documents are listed in the continuation of Box C.　　　☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | "&" | document member of the same patent family |

| Date of the actual completion of the international search | Date of mailing of the international search report |
|---|---|
| 04/08/2023 | **(10/08/2023)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | I. Abad Gurumeta<br><br>Telephone No. 913495337 |

Form PCT/ISA/210 (second sheet) (July 2022)

## INTERNATIONAL SEARCH REPORT

Information on patent family members

International application No.

PCT/ES2022/070777

| Patent document cited in the search report | Publication date | Patent family member(s) | Publication date |
|---|---|---|---|
| CN110470600 A | 19.11.2019 | NONE | |
| US2008262739 A1 | 23.10.2008 | US7702473 B2 | 20.04.2010 |
| US2008011061 A1 | 17.01.2008 | US2008295615 A1<br>US7542855 B2<br>US7437248 B2 | 04.12.2008<br>02.06.2009<br>14.10.2008 |
| CN101573302 A | 04.11.2009 | TW200918467 A<br>CA2666331 A1<br>AU2007312940 A1<br>WO2008046139 A1<br>WO2008046139 A8 | 01.05.2009<br>24.04.2008<br>24.04.2008<br>24.04.2008<br>03.07.2008 |

Form PCT/ISA/210 (patent family annex) (July 2022)

# EP 4 632 373 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ZAIWEN WEN et al.** *Math. Prog. Comp*, 2012, vol. 4, 333-361